# EUROPEAN PATENT SPECIFICATION

(11) **EP 4 282 358 B1**
(45) Date of publication and mention of the grant of the patent: **28.05.2025**
(21) Application number: 23170314.1
(22) Date of filing: 27.04.2023
(51) Int. Cl.: A61B 17/70, A61B 5/00, A61B 5/01, A61B 5/07, A61B 5/1495, A61B 5/024, A61B 5/11, A61B 17/00, A61B 90/00

(54) **LOAD SENSING ASSEMBLY FOR A SPINAL IMPLANT**
LASTABGRIFFS-BAUGRUPPE FÜR EIN WIRBELSÄULENIMPLANTAT
ASSEMBLAGE DE DÉTECTION DE CHARGE POUR IMPLANT SPINAL

(30) Priority: 18.05.2022 US 202217747022
(43) Date of publication of application: 29.11.2023
(73) Proprietor: Warsaw Orthopedic, Inc., Warsaw, IN 46582 (US)
(72) Inventor: METCALF, JR., Newton H., Memphis, TN (US); SIBY KURIAN, Arjun, Memphis, TN (US); JOHNSON, Chris E., Germantown, TN (US); CALCUTT, Simon, Melbourn (GB); WYLDE, George, Melbourn (GB); POOLEY, David Martin, Cambridge (GB); LINTERN, Richard David, Cambridge (GB)
(74) Representative: Viering, Jentschura & Partner mbB Patent- und Rechtsanwälte

(56) References cited:
- US-A1- 2020 022 740
- US-A1- 2021 153 909

## Description

### TECHNICAL FIELD

The present disclosure generally relates to mechanical and electrical sensor assemblies and antenna designs for implant devices, and more particularly to implant systems including a set screw that may be used to secure a connection between a longitudinal rod and a pedicle screw, hook or other connector which may be used to treat various spinal disorders. In particular, the present invention relates to a load sensing assembly for a spinal implant.

### BACKGROUND

Treatment of spinal disorders, such as degenerative disc disease, disc herniations, scoliosis or other curvature abnormalities, and fractures, often requires surgical treatments. For example, spinal fusion may be used to limit motion between vertebral members. As another example, implants may be used to preserve motion between vertebral members.

Surgical treatment typically involves the use of longitudinal members, such as spinal rods. Longitudinal members may be attached to the exterior of two or more vertebral members to assist with the treatment of a spinal disorder. Longitudinal members may provide a stable, rigid column that helps bones to fuse, and may redirect stresses over a wider area away from a damaged or defective region. Also, rigid longitudinal members may help in spinal alignment.

Screw assemblies may be used to connect a longitudinal member to a vertebral member. A screw assembly may include a pedicle screw, hook, or other connector and/or a set screw, among other components. A pedicle screw can be placed in, above and/or below vertebral members that were fused, and a longitudinal member can be used to connect the pedicle screws which inhibits or controls movement. A set screw can be used to secure the connection of a longitudinal member and a pedicle screw, hook or other connector. However, the connection force and continued integrity of the connection between a longitudinal member and a pedicle screw or other connector can be challenging to monitor during and after implantation. In addition, it is difficult to monitor that an appropriate force is maintained between a set screw and a longitudinal member. Conventional load assemblies and/or screw assemblies are not capable of sensing and wirelessly transmitting the connection force between a longitudinal rod and a pedicle screw installed within a patient. Furthermore, they cannot continuously monitor and maintain a secure connection on relatively long time frames.

A load sensing assembly for a spinal implant according to the preamble of claim 1 is, e.g., known from US 2021 / 153 909 A1.

US 2020 / 022 740 A1 discloses a load sensing assembly for a spinal implant.

### SUMMARY

The invention provides a load sensing assembly for a spinal implant according to claim 1. Further embodiments are described in the dependent claims.

Associated surgical methods are also described herein to aid understanding the invention. These methods do not form part of the claimed invention.

### BRIEF DESCRIPTION OF THE DRAWINGS

The embodiments which form part of the invention are illustrated in the figures 9-16.

The examples shown in the other figures do not form part of the invention but represent background art that is useful for understanding the invention.
FIG. 1 illustrates an example anchoring assembly and longitudinal member according to an example.
FIG. 2 illustrates an example exploded view of a screw assembly and longitudinal member according to an example.
FIG. 3A illustrates an exploded view of an example set screw according to an example.
FIG. 3B illustrates an example set screw according to an example.
FIG. 4 illustrates a cross section drawing of an example set screw.
FIG. 5A illustrates a cross section drawing of a body portion of a set screw.
FIG. 5B illustrates a cross section drawing of a cover portion of a set screw.
FIG. 6A illustrates a side view of an example set screw at a first assembly stage.
FIG. 6B illustrates a bottom view of an example cover portion after being welded to the example set screw of FIG. 6A.
FIG. 7 illustrates a side view of an example set screw showing the interior electronic components in skeleton outlining.
FIG. 8 illustrates an example of a surgical site monitoring system according to an example.
FIG. 9 illustrates a partial cut out perspective view of an embodiment of a digital set screw having a lateral antenna and ferrite core according to the invention.
FIG. 10A illustrates a perspective view of a ferrite core for use with the embodiment of FIG. 9.
FIG. 10B illustrates a perspective view of an antenna, including a copper winding surrounding at least a portion of the ferrite core of FIG. 10A.
FIG. 11A illustrates a first side view of the antenna of FIG. 10B.
FIG. 11B illustrates a second side view of the antenna of FIG. 10B.
FIG. 12 illustrates a cross section view of an embodiment of a digital set screw.
FIG. 13 illustrates a perspective view of a digital set screw.
FIG. 14 illustrates a cross section drawing of another digital set screw embodiment.
FIG. 15 illustrates a cross-section drawing of another digital set screw embodiment.
FIG. 16 illustrates a perspective view of a spline drive digital set screw.

### DETAILED DESCRIPTION

References to "embodiments" throughout the description which are not under the scope of the appended claims merely represent possible exemplary executions and are therefore not part of the present invention

The exemplary embodiments of the surgical system and related methods of use (not claimed) disclosed are discussed in terms of medical devices for the treatment of musculoskeletal disorders and more particularly, in terms of a vertebral fixation screws, including for example pedicle screws, as well as hooks, cross connectors, offset connectors and related systems for use during various spinal procedures or other orthopedic procedures and that may be used in conjunction with other devices and instruments related to spinal treatment, such as rods, wires, plates, intervertebral implants, and other spinal or orthopedic implants, insertion instruments, specialized instruments such as, for example, delivery devices (including various types of cannula) for the delivery of these various spinal or other implants to the vertebra or other areas within a patient in various directions, and/or a method or methods for treating a spine, such as open procedures, mini-open procedures, or minimally invasive procedures. Exemplary prior art devices that may be modified to include the various embodiments of load sensing systems include, for example, U.S. Patents 6,485,491 and 8,057,519.

The present disclosure may be understood more readily by reference to the following detailed description of the embodiments taken in connection with the accompanying drawing figures, which form a part of this disclosure.

**In** some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior". Generally, similar spatial references of different aspects or components indicate similar spatial orientation and/or positioning, i.e., that each "first end" is situated on or directed towards the same end of the device. Further, the use of various spatial terminology herein should not be interpreted to limit the various insertion techniques or orientations of the implant relative to the positions in the spine.

The following discussion includes a description of a vertebral pedicle screw system and related components and methods of employing the vertebral pedicle screw in accordance with the principles of the present disclosure. Reference is made in detail to the exemplary embodiments of the present disclosure, which are illustrated in the accompanying figures.

The components of the vertebral pedicle screw system described herein can be fabricated from biologically acceptable materials suitable for medical applications, including metals, synthetic polymers, ceramics and bone material and/or their composites. Some ceramics may be formed of Zirconia 3Y-TZP and/or a Zirconia toughed alumina (ZTA), for example. Additionally, various the components of the vertebral pedicle screw system, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL^{®}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations.

Various components of the vertebral pedicle screw system may be formed or constructed material composites, including the above materials, to achieve various desired characteristics such as strength, rigidity, elasticity, compliance, biomechanical performance, durability and radiolucency or imaging preference. The components of the present vertebral pedicle screw system, individually or collectively, may also be fabricated from a heterogeneous material such as a combination of two or more of the above-described materials. The components of the vertebral pedicle screw system may be monolithically formed, integrally connected or include fastening elements and/or instruments, as described herein. The components of the vertebral pedicle screw system may be formed using a variety of subtractive and additive manufacturing techniques, including, but not limited to machining, milling, extruding, molding, 3D-printing, sintering, coating, vapor deposition, and laser/beam melting. Furthermore, various components of the vertebral pedicle screw system may be coated or treated with a variety of additives or coatings to improve biocompatibility, bone growth promotion or other features. To the extent the plate is entirely or partially radiolucent, it may further include radiographic markers made, for example of metallic pins, at one or both ends, on each corner of the ends, and/or along the length of the implant in various locations including near the center of the assembly.

The vertebral pedicle screw system may be employed, for example, with a minimally invasive procedure, including percutaneous techniques, mini-open and open surgical techniques to deliver and introduce instrumentation and/or one or more spinal implants at a surgical site within a body of a patient, for example, a section of a spine. In some embodiments, the vertebral pedicle screw system may be employed with surgical procedures, as described herein, and/or, for example, corpectomy, discectomy, fusion and/or fixation treatments that employ spinal implants to restore the mechanical support function of vertebrae. In some embodiments, the pedicle screw system may be employed with surgical approaches, including but not limited to: anterior lumbar interbody fusion (ALIF), direct lateral interbody fusion (DLIF), oblique lateral lumbar interbody fusion (OLLIF), oblique lateral interbody fusion (OLIF), transforaminal lumbar Interbody fusion (TLIF), posterior lumbar Interbody fusion (PLIF), various types of posterior or anterior fusion procedures, and any fusion procedure in any portion of the spinal column (sacral, lumbar, thoracic, and cervical, for example).

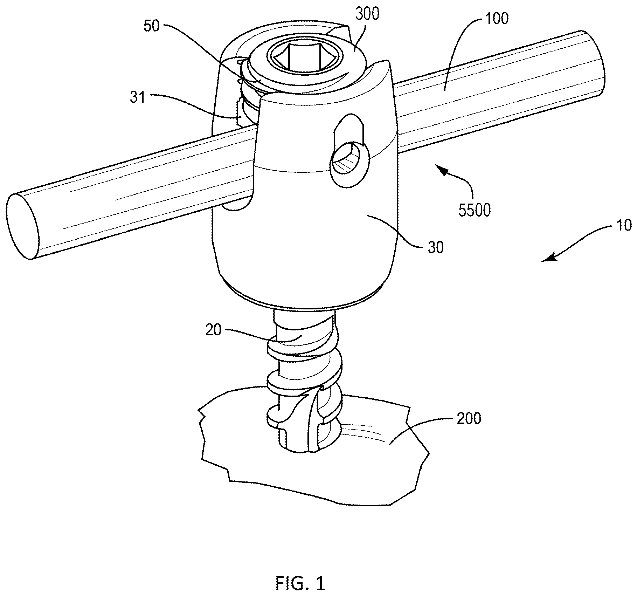

Referring generally to FIGS. 1-3, an example set screw assembly and anchoring assembly is illustrated. FIG. 1 may illustrate an example anchoring assembly and longitudinal member according to an embodiment. As illustrated in FIG. 1, an anchoring assembly may include a screw 20 and an anchoring member 30. The screw 20 may have an elongated shape with a first end mounted within a vertebral member 200 and a second end extending outward above the vertebral member 200. The anchoring member 30 may be configured to operatively connect to the second end of the screw 20 and may be movably connected to the screw 20 to accommodate the longitudinal member 100 positioned at various angular positions. The anchoring member 30 may include a channel 31 sized to receive the longitudinal member 100. A set screw 50 may attach to the anchoring member 30 to capture the longitudinal member 100 within the channel 31.

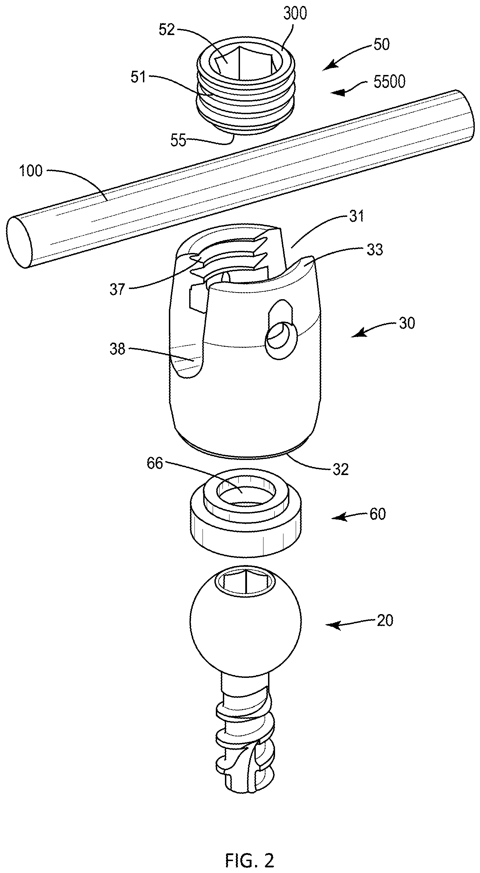

FIG. 2 illustrates an example exploded view of a screw assembly and longitudinal member according to an embodiment. As shown by FIG. 2, anchoring member 30 provides a connection between the screw 20 and longitudinal member 100. Anchoring member 30 includes a first end 32 that faces towards the vertebral member 200, and a second end 33 that faces away. A chamber may be positioned between the first and second ends 32, 33 and may be sized to receive at least a portion of the screw 20. In various embodiments, a first end 32 may be considered a base portion of an anchoring member 30, and a second end 33 may be considered a head portion of an anchoring member.

The second end 33 of the anchoring member 30 may include a channel 31 sized to receive the longitudinal member 100. Channel 31 terminates at a lower edge 38 that may include a curved shape to approximate the longitudinal member 100. Threads 37 may be positioned towards the second end 33 to engage with the set screw 50. In one embodiment as illustrated in FIG. 2, the threads 37 are positioned on the interior of the anchoring member 30 facing towards the channel 31. In another embodiment, the threads 37 may be on the exterior of the anchoring member 30. An interior of the anchoring member 30 may be open between the first and second ends 32, 33.

In various embodiments, an anchoring member 30 may include a washer 60. A washer 60 may be generally cylindrical and may have a hole 66 there through. As illustrated by FIG. 1 a washer 60 may be positioned near a first end 32 of an anchoring member 30. A screw 20 may engage with an anchoring member 30 via positioning through the hole 66 of a washer 60. A washer 60 may include recessed portions which may be configured to accommodate placement of a longitudinal member 100 therein. The use of a washer 60 in connection with an anchoring member 30 may help minimize misalignment of the longitudinal member within the anchoring member.

In an embodiment, set screw 50 attaches to the anchoring member 30 and captures the longitudinal member 100 within the channel 31. Set screw 50 may include an antenna 300, which will be explained in further detail below. As illustrated in FIG. 2, the set screw 50 may be sized to fit within the interior of the channel 31 and include exterior threads 51 that engage threads 37 on the anchoring member 30. Additionally, a cover portion 55 may contact longitudinal member 100 when the set screw 50 is rotatably positioned into a closed contact position above longitudinal member 100 and within anchoring member 30. A driving feature 52 may be positioned on a top side to receive a tool during engagement with the anchoring member 30, e.g., a screwdriver or the like having a corresponding head. In some embodiments, the set screw 50 may be mounted on an exterior of the anchoring member 30. Set screw 50 may include a central opening that partially extends into set screw 50 from drive feature 52 towards the second end 33. For example, the drive feature 52 may be a recessed portion having a covered bottom portion and circumferential side walls such that the drive feature 52 portion of set screw 50 does not fully extend through the set screw 50 from the top to the bottom. Threads 51 are positioned on an outside surface of the set screw 50 and engage with the threads 37 on the anchoring member 30. The set screw 50 and anchoring member 30 may be constructed for the top side of the set screw 50 to be flush with or recessed within the second end 33 when mounted with the anchoring member 30. For example, a common plane crossing over a top surface of antenna 300 and a top surface of second end 33.

Although FIGS. 1 and 2 illustrate an exemplary multi-axial tulip-head pedicle screw it shall be understood that other types of anchoring members may be used within the scope of this disclosure. For example, fixed head screws or screws having differently shaped heads may be used. As another example, a hook member, a cross-link connector, an offset connector, or a hybrid hook-screw member may be used as well.

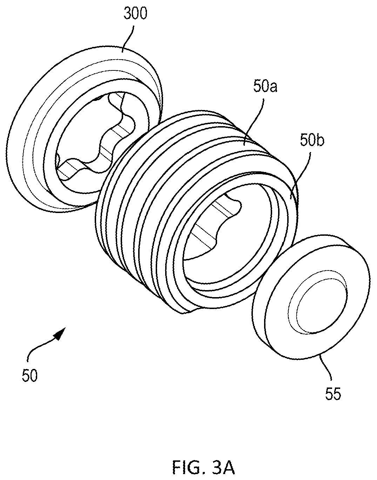

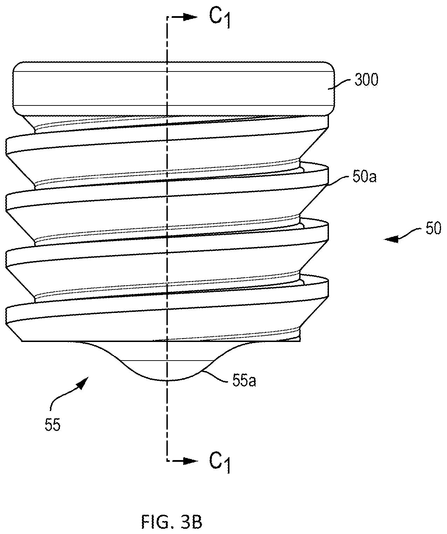

FIG. 3A illustrates an exploded view of an example set screw 55, and FIG. 3B illustrates set screw 50 after being fully assembled according to an embodiment. As illustrated, set screw 50 may include an antenna 300, a body portion 50a, and a cover portion 55. Body portion 50a may include an outside surface with threads and a lower body cavity portion 50b defined by a depth and interior radius that corresponds to a depth and exterior radius of cover portion 55. For example, cover portion 55 may be dimensioned to fit inside at least a portion of body cavity portion 50b such that only a protrusion 55a extends from a lowermost surface of body portion 50a. Protrusion 55a may directly contact longitudinal rod 100 when set screw 50 is fully secured within anchoring member 30.

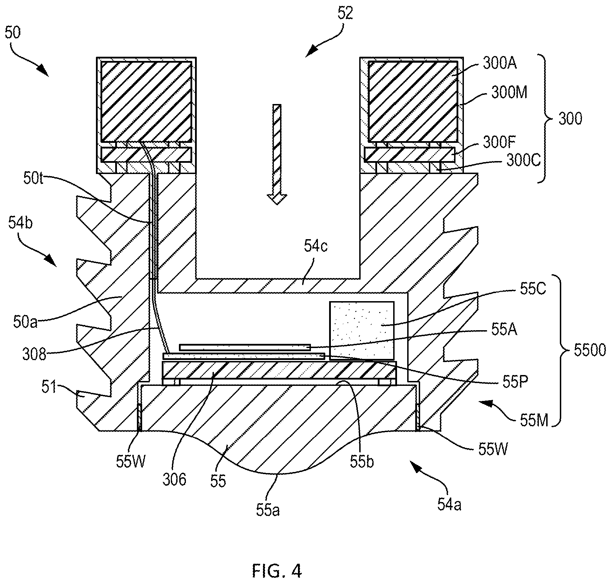

FIG. 4 illustrates a cross section drawing of an example set screw 50 taken along line C₁-C₁ of FIG. 3B. In the example illustration, antenna 300 may be disposed on a top portion of main body 50a and cover portion 55 may be disposed in a lower cover cavity 54a of main body 50a. In example embodiments, antenna 300, may be a radio frequency identification (RFID) coil, a near field-communication (NFC) antenna or other short-range communication transmitter and/or receiver. Antenna 300 may include an axisymmetric coil 300a stacked on a ferrite base 300f and a carrier 300c. The axisymmetric coil 300a, ferrite 300f, and carrier 300c may be surrounded by an overmold 300m. An example overmold 300m may be an insulator material, such as a thermoplastic material like Polyether ether ketone (PEEK). In some embodiments, antenna 300 may be fixedly coupled to a top portion of set screw 50 and in other embodiments, antenna 300 may be removably coupled to a top portion of set screw 50, e.g., by mechanical means such as corresponding threads or snap locking features.

In an example embodiment, set screw 50 may include a drive feature 52 that passes through antenna 300 and into a cavity of set screw 50 that is defined by interior sidewalls of set screw 50 and a bottom sidewall 54c. Antenna 300 may also include a flexible electronics component, such as, for example, a flex circuit or one or more electrical circuits operably connected to the electronics components 5500 via a connecting member 308. For instance, as shown in FIG. 4, the connecting member 308 may be connected to both the antenna 300 and the electronics components 5500. The connecting member 308 may be positioned perpendicularly to both the antenna 300 and the electronics components 5500. In an example embodiment, connecting member 308 may pass from antenna 300 through a through hole 50t passing through main body 50a and into electronics cavity 54b for housing electronics components 5500. In an example embodiment, through hole 50t may be filled with an insulating material, for example the same or substantially same material as the overmold 300m. However, it shall be understood that through hole 50t may be filed with any suitable material that is effective at sealing through hole 50t, e.g., an epoxy or the like.

Example, electronics components 5500 may include a series of electronic components in electrical communication with one another. For example, a mainboard or other suitable printed circuit board (PCB) 55p may be electrically connected to an application specific integrated circuit (ASIC) 55a, a charge storage capacitor 55c, and various mechanical electrical sensors or micro electromechanical systems (MEMs) 55m. Example MEMs 55m may include a strain gauge, and/or a temperature gauge. However, other MEMs sensors may be incorporated in other embodiments depending on the particular use case. In some embodiments, electronics components 5500 may be an prepackaged self-contained unit that is attached to cover 55 by, e.g., adhesive, chemical, mechanical or cement bonding. Additionally, electronics components 5500 may include a non-transitory data store (not illustrated) according to an embodiment, e.g., a memory cell such as a solid state memory cell or the like. The non-transitory memory data store may store information and/or data from various MEMs sensors 55m, for example. A non-transitory data store may be used to store various information. For example, one or more measurements of a strain gauge 306 may be stored in memory. As another example, a unique identifier associated with a load sensing assembly, a component thereof, or a set screw 50 may be stored in memory. Additional and/or alternate information or types of information may be stored as is consistent with this disclosure. Additionally, in some embodiments, electronics components 5500 may be coated in a material to prevent and/or suppress corrosion, e.g., a conformal coating, an epoxy coating, aerosol coating, or the like.

In various embodiments, electronics components 5500 may be fixedly coupled to cover portion 55 and have a connecting terminal or connecting portion 308 extending therefrom. The connecting terminal or connecting portion 308 may be suitably connected to a lead wire extending from antenna 300. For example, as shown in FIG. 4, electronics components 5500 are in electrical communication with antenna 300 by way of connecting portion 308. In various embodiments, connecting member 308, antenna 300, and/or electronics components 5500 may be constructed integrally or may be separately constructed and attached together in any suitable manner, such as for example by adhesive, chemical, mechanical or cement bonding.

In at least one embodiment, electronics components 5500 may be configured as a load sensing assembly. A load sensing assembly may include one or more electronics components 304 and/or a strain gauge 306, such as for example a silicon strain gauge. A strain gauge 306 may be a device that measures strain on an object. For instance, a strain gauge 306 may measure a force between a set screw and a longitudinal member when the set screw is engaged with an anchoring member. A strain gauge 306 may include one or more sensors or sensor nodes that measure strain, force, resistance, deflection, load and/or the like.

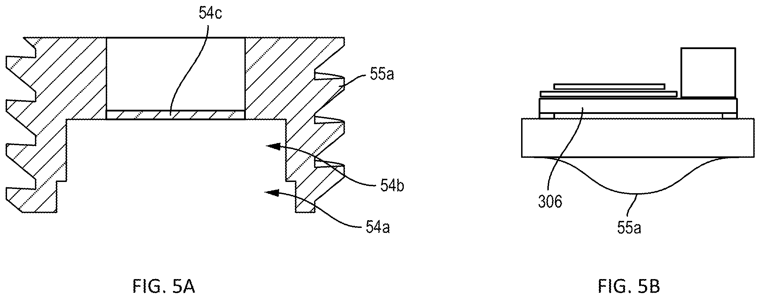

As illustrated in FIGS. 5A and 5B, a load sensing assembly may be configured to be coupled to a set screw 50. For example, cover 55 including electronics components 5500 may be coupled to set screw 50. In at least one embodiment, cover 55 including pre-installed electronics components 5500 are installed/inserted into the bottom portion of main body 50a in the corresponding lower cover cavity 54a. In at least one embodiment, lower cover cavity 54a may be dimensioned such that exterior side wall portions of cover 55 directly contact interior sidewall portions of cover cavity 54a. For example, lower cover cavity 54a may be defined by a radius of a first circle that corresponds to a radius of cover 55. However, in other embodiments, cover 55 may be oval shaped, hexagonal, or square and in those embodiments cover cavity 54a has a corresponding geometry imparting the same, substantially the same, or similar functional arrangement. Additionally, by installing cover 55 including pre-installed electronics components 5500 into a body portion of set screw 50 the electronics components 5500 are housed with electronics cavity 54b. Electronics cavity may open up to lower cover cavity 54a and may be enclosed at a top portion corresponding to the bottom sidewall 54c of drive interface 52. For example, bottom sidewall 54c may also function as a top surface, or ceiling, of electronics cavity 54b. It shall be understood that the relative position of bottom sidewall 54c may be lowered to accommodate different drivers interactive with drive feature 52 or may be raised to accommodate differently sized electronics components 5500. In an embodiment, the electronics cavity 54b may have a height of about twice the height of cover cavity 54a. Cover cavity 54a may be defined by a radius of a first circle in the horizontal direction and electronics cavity 54b may be defined by the radius of a second circle in the horizontal direction. In at least one embodiment, the radius of the first circle (corresponding to the cover cavity 54a) may be greater than the radius of the second circle (corresponding to the electronics cavity 54b). For example, an inset step may be provided where the cover cavity 54a and electronics cavity 54b meet one another relative to the main body 50a.

Consistent with previous disclosure, a strain gauge 306 may be operably connected, for example by adhesive, cement, mechanical or chemical bonding, to the electronics components 5500. For instance, a strain gauge 306 may be operably connected to the electronics components 5500 via the bottom surface 312 of the electronics components 5500. A strain gauge 306 may be connected to an inside surface 55b of cover 55 in any suitable manner including, without limitation, via an adhesive bonding agent. Strain gauge 306 may be situated in this way to detect strain in a curved contact portion of 55a acting against longitudinal rod 100 (see FIGS. 1 and 2).

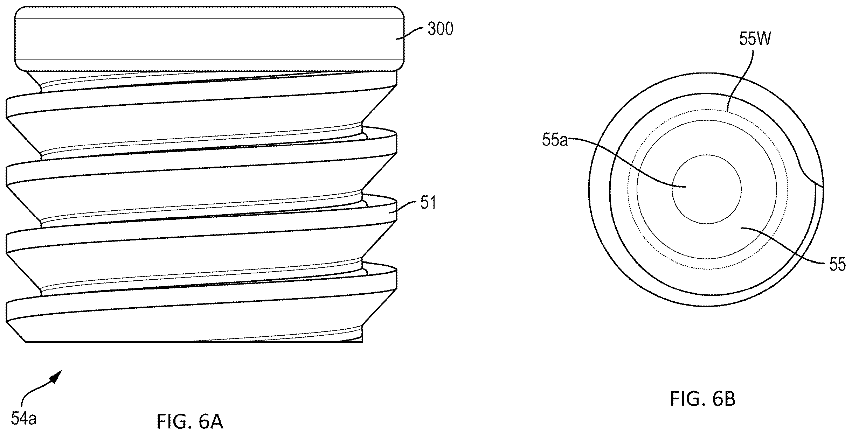

FIG. 6A illustrates an example set screw 50 at a first assembly stage with a pre-installed antenna 300. FIG. 6B illustrates a bottom view of an example set screw 50 after cover portion 55 has been fixedly attached to example set screw 50 of FIG. 6A. In at least one embodiment, cover portion 55 is fixedly attached to set screw 50 by a continuous weld 55w (see also FIG. 4). The continuous weld 55w may be any type of weld, for example a fillet weld or a butt joint weld. The continuous weld may be performed by any method, for example laser welding, gas welding, electron beam welding, etc. In some embodiments, a series of tack welds (not illustrated) may be performed at discrete intervals in substantially the same way and at substantially the same locations. At least one advantage to fixedly coupling cover portion 55 as previously disclosed is that electronics components 5500 may be securely protected by virtue of being in a fully sealed cavity, for example an airtight and water tight cavity, i.e., a hermetically sealed cavity.

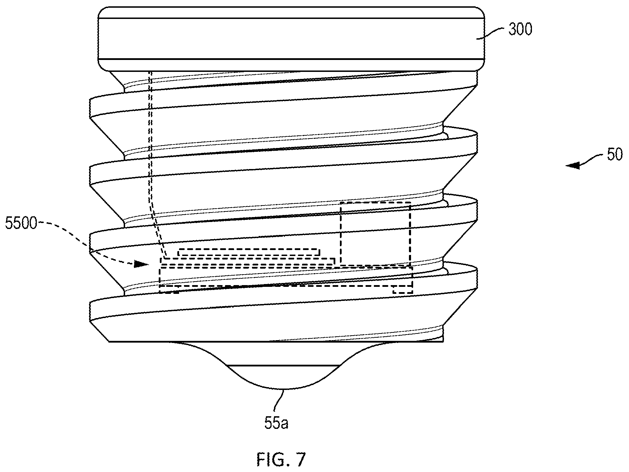

FIG. 7 illustrates a side view of an example set screw 50 showing the interior electronic components 5500 in skeleton outlining. As illustrated in FIG. 7, the antenna 300 may circumferentially surround at least a portion of the exterior of the set screw 50. For example, antenna 300 may cover, at least partially, a top surface of set screw 50. In some embodiments, antenna 300 may cover the entire top surface (uppermost surface) of set screw 50. For example, antenna 300 may circumferentially surround a top surface of set screw 50. In other embodiments, the antenna 300 may be positioned at least partially inside of the central opening of a set screw (not illustrated). For example still, the antenna may be located, at least in part, proximate to a top portion of set screw 50. Consistent with the above disclosure, in certain embodiments, the strain gauge 306 may be connected to cover 55 such that it is positioned to measure a force between the set screw 55 and a longitudinal rod 100 (see FIGS. 1 and 2) when the set screw 50 engages with an anchoring member, for example protrusion 55a. Additionally or alternatively, other MEMs sensors, such as a temperature gauge for example, may be positioned to discern a body temperature of a patient in the same, similar, or substantially the same way. In at least one embodiment, a temperature sensor is positioned to discern a body temperature of a patient in the region of the longitudinal rod, and in others a temperature sensor is positioned to discern a body temperature of a patient in a region adjacent a threaded portion of set screw 51 that is directly exposed to patient tissue (i.e., not a portion contacting longitudinal rod 100).

In various embodiments, one or more measurements obtained by strain gauge 306 may be stored by an integrated circuit of a corresponding load sensing assembly such as, for example, in non-transitory computer readable memory as disclosed above. In turn, antenna 300 and/or electronics components 5500 may be interrogated by a reader. For instance, an RFID chip may be read by an RFID reader. As another example, an NFC chip may be read by or may otherwise communicate with an NFC reader or other NFC-enabled device. In other embodiments, a custom protocol may be used, for example a 125kHz inductive link. Example readers may include at least one antenna for receiving and/or transmitting data with antenna 300 of set screw 50, a central processing unit CPU, and a non-transitory computer readable medium (such as a memory unit or memory cell storing programmable computer implemented instructions). In at least one embodiment, an electromagnetic reader (first reader) may transmit electromagnetic energy to set screw 50 to power electronic components 5500 and an RFID reader or an NFC reader (second reader) may be used separately to read, acquire, and/or interpret data received from antenna 300. A reader may interrogate an integrated circuit when in a certain proximity to the integrated circuit. In certain embodiments, a reader may interrogate an integrated circuit that has been implanted into a patient as part of a set screw or anchoring member assembly. In other embodiments, an integrated circuit may communicate with a reader or other electronic device without being interrogated.

An integrated circuit of electronics components 5500 may transmit one or more measurements to the reader. This transmission may occur in response to being interrogated by the reader, or the transmission may be initiated by the integrated circuit. The reader may receive the transmitted measurements, and may cause at least a portion of the measurements to be displayed to a user. For instance, a physician may use a reader to interrogate an RFID chip of a patient's implant. The reader may include a display, or may be in communication with a display device, which may display at least a portion of the measurements received from the RFID chip.

Electronic components 5500 may include a passive integrated circuit. An example passive integrated circuit may refer to an arrangement where electronic components 5500 do not include an internal power source. For example, electronic components 5500 may be powered by energy transmitted from a reader. With respect to electronic components 5500 having a passive integrated circuit, the passive integrated circuit may not transmit information until interrogated by a reader. For example, a reader may transmit electromagnetic energy directed at the passive integrated circuit to wirelessly power the passive integrated circuit. At least two advantages of using an integrated circuit that does not include a battery or require a battery is reliability, and reduction in space within the cavity 54b that houses the electronic components 5500 forming a passive integrated circuit.

In various embodiments, one or more sensors of electronic components 5500 may transmit information by directly modulating a reflected signal, such as an RF signal. The strain gauge 306 sensors may form a Wireless Passive Sensor Network (WPSN), which may utilize modulated backscattering (MB) as a communication technique. External power sources, such as, for example, an RF reader or other reader, may supply a WPSN with energy. The sensor(s) of the WPSN may transmit data by modulating the incident signal from a power source by switching its antenna impedance.

In another embodiment, an integrated circuit may be active, meaning that the chip is battery-powered and capable of broadcasting its own signal. An active integrated circuit may transmit information in response to be interrogated by a reader, but also on its own without being interrogated. For instance, an active integrated circuit may broadcast a signal that contains certain information such as, for example, one or more measurements gathered by an associated strain gauge. An active integrated circuit may continuously broadcast a signal, or it may periodically broadcast a signal. Power may come from any number of sources, including, for example, thin film batteries with or without encapsulation or piezo electronics.

One or more measurements received from a load sensing assembly may be used to make determinations of the condition of a spinal implant and/or treatment of a spinal disorder. For instance, proper placement of a longitudinal member, set screw and/or anchoring member may result in an acceptable range of force measurements collected by a strain gauge of a load sensing assembly. Measurements outside of this range may indicate a problem with the placement or positioning of a longitudinal member, set screw and/or anchoring member such as, for example, loosening of a set screw and/or anchoring member, longitudinal member failure, construct failure, yield or fracture/breakage, improper torque, breakage of the bone segment or portion, the occurrence of fusion or amount of fusion, and/or the like. In these instances, the reader may contain a range of pre-determined acceptable values corresponding to the strain gauge 306 and/or other MEMs sensors. If the actual measured reading of the strain gauge 306 and/or other MEMs sensors falls outside of the range, the reader may notify an end user, a hospital management system, and/or the patient. For example, a patient may continuously or regularly monitor the actual measured readings of set screw 50 on an outpatient basis with a reader. In some embodiments, a reader may be configured to relay information received from antenna 3000 to a secondary processing component such as an external display, computer, server, hospital management system, or other type of data processing equipment. The secondary processing component may process information received by the reader from antenna 300 via a processor, controller, and memory configured to execute programmable computer implemented instructions. In this way, disclosed systems increase the likelihood that a patient can detect a malfunction, such as loosing of a set screw 50 and/or interbody system (see FIGS. 1 and 2), before catastrophic failure.

One or more tools or instruments may include a reader which may be used to gather information from one or more integrated circuits of electronic components 5500 during or in connection with a procedure. For instance, a torque tool (not illustrated) may be used to loosen or tighten set screw 50. A torque tool may include a reader, or may be in communication with a reader, such that a user of the torque tool is able to obtain, in substantially real time, one or more measurements relating to the set screw 50 and longitudinal rod 100 placement that are measured by a strain gauge 306 of a load sensing assembly of the set screw 50 via the tool. For instance, as a user is applying torque to a set screw 50, the user may see one or more force measurements between the set screw 50 and the longitudinal member in order to determine that the positioning of the set screw 50 and/or longitudinal member is correct and that the proper force is being maintained. In certain embodiments, a tool or instrument may include a display device (not illustrated) on which one or more measurements may be displayed. In other embodiments, a tool or instrument may be in communication with a display device (not illustrated), and may transmit one or more measurements for display on the display device via a communications network.

In some embodiments, an electronic device, such as a reader or an electronic device in communication with a reader (not illustrated), may compare one or more measurements obtained from an integrated circuit to one or more acceptable value ranges. If one or more of the measurements are outside of an applicable value range, the electronic device may cause a notification to be made. For instance, an electronic device may generate an alert for a user, and cause the alert to be displayed to the user via a display device. Additionally or alternatively, an electronic device may send an alert to a user such as via an email message, a text message, a notification, or otherwise.

An integrated circuit of electronics components 5500 may store a unique identifier associated with the components to which the load sensing assembly corresponds. For example, an integrated circuit of electronics components 5500 for a set screw 50 may store a unique identifier associated with the set screw 50 and/or cover 55. For example, when a reader interrogates an integrated circuit, the integrated circuit may transmit a unique identifier for a component that is stored by the integrated circuit to the reader. Having access to a unique identifier for a component may help a user ascertain whether the measurements that are being obtained are associated with the component of interest. Also, having access to a unique identifier for a component may help a user take inventory of one or more components. For instance, after spinal surgery, a physician or other health care professional may use a reader to confirm that all of the set screws and anchoring members allocated for the procedure have been used and are positioned in a patient. This may also help with the detection and verification of lost screws in a patient's body.

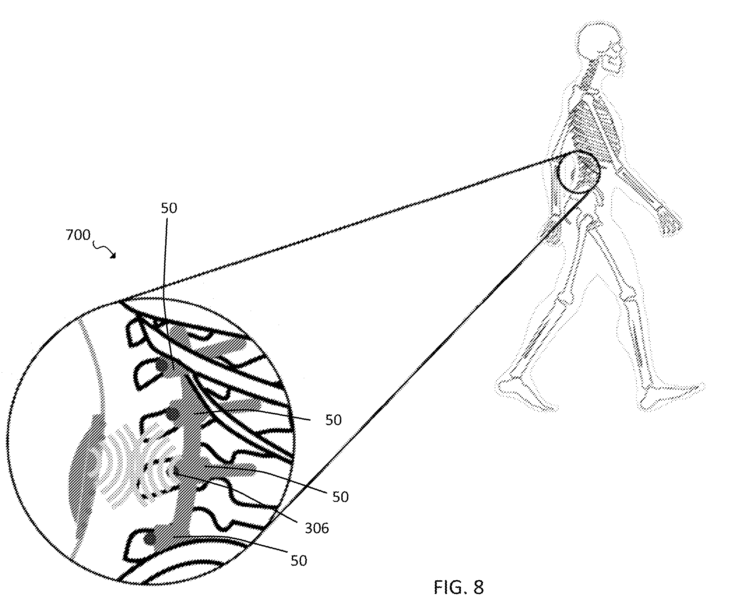

FIG.8 illustrates an example of a surgical site (SS) monitoring system 700 that may utilize example set screws 50 disclosed herein. In some embodiments, the SS monitoring system 700 may be a surgical site load monitoring system (at least those set screws 50 utilizing a strain gauge 306) and/or an infection monitoring system (at least those set screws 50 utilizing a temperature sensor).

In one or more embodiments, the SS monitoring system 700 may include an array of set screws 50, in which one or more of the set screws 50 have a have any type of MEMs sensor as previously disclosed. For the cases in which the SS monitoring system 700 includes an array of set screws 50 having various MEMs sensors, the received data from the one or more MEMs sensors of set screws 50 may be compared to one another to diagnose the quality of the surgical procedure, the integrity of the implant, and/or an infection at the surgical site.

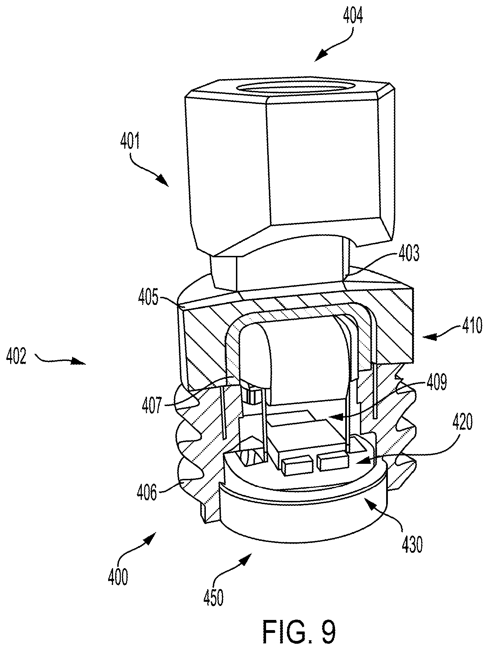

FIGS. 9-16 illustrate an embodiment of a digital set screw 400 having a lateral antenna design. Digital set screw 400 may include the same, similar, or substantially the same features and functionality as the other embodiments of this disclosure and the co-related applications. For example, features from those embodiments may be incorporated into digital set screw 400 and vice versa unless the context clearly indicates otherwise.

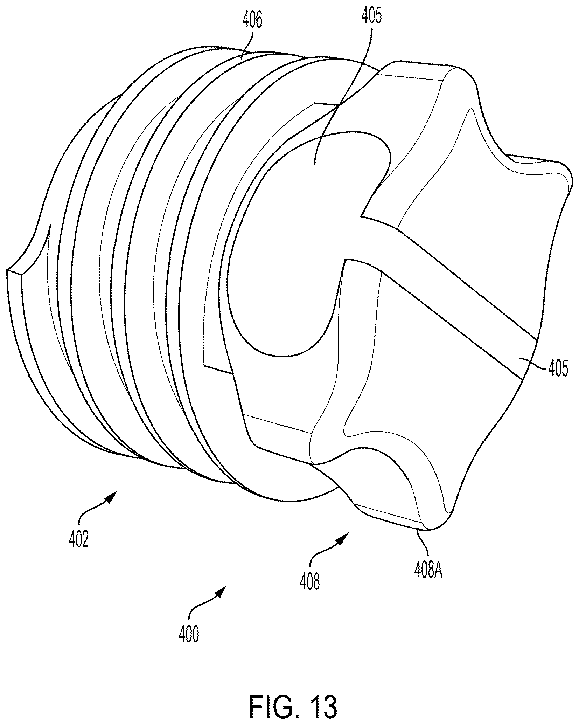

FIG. 9 illustrates a partial cut out perspective view of a digital set screw 400 having a break off portion 401 and a remaining set screw portion 402. In various embodiments, digital set screw portion 402 may include a hermetically sealed cavity 409 housing an antenna 410, microelectronics 420, and sensors 430, for example. Consistent with this disclosure and the incorporated disclosures, sensors 430 may be any type of sensor, e.g., strain gauges, temperature gauges, etc. Additionally, sensors 430 may be arranged in any pattern, e.g., single, centered, distributed into quadrants, various patterns, etc. Breakoff portion 401 may include a drive feature 404 taking the shape of a hexalobe aperture, for example. Other drive features having different shapes are also contemplated, for example hexagonal, polygonal, torx, square head, spline, etc. In various embodiments, breakoff portion 401 may be configured to shear off from set screw portion 402 at a break off location or fracture surface 403 at a predetermined torque ensuring that such internal stresses are suppressed and/or do not interfere with the hermetic seal and electronics components within cavity 409, for example. In various embodiments, digital set screw 400 and breakoff portion may be compatible with the Solera Spinal Systems sold by Medtronic of Minneapolis Minnesota. In the disclosed embodiment, once breakoff portion 401 is broken off, the remaining set screw portion 402 may have a torx head or a nut style head taking any suitable shape such that the set screw portion 402 may be still be driven and/or rotated absent the breakoff portion 401, e.g., a Torx 50. For example, as shown in FIG. 13, the breakoff portion 401 is removed and the remaining set screw portion 402 includes a torx head portion 403.

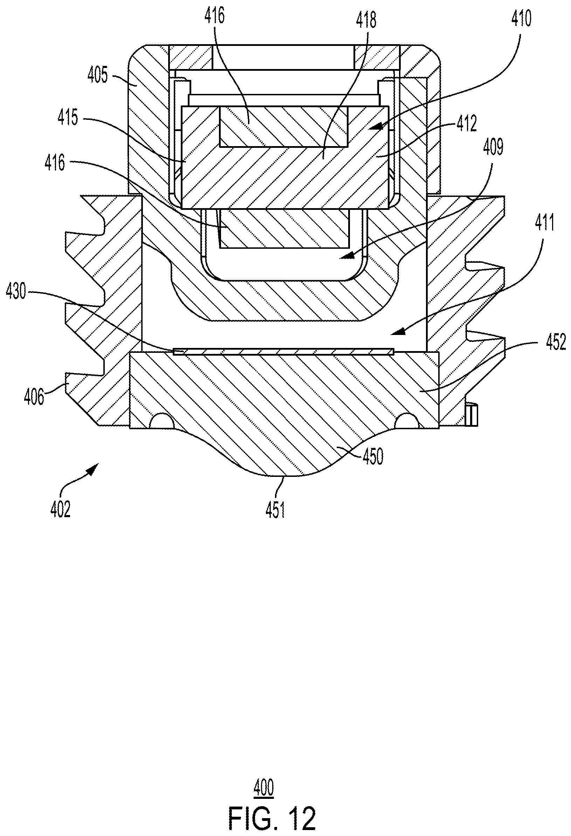

In some embodiments, set screw portion 402 may include an over-molding 405 and a threaded portion 406. In various embodiments, over-molding 405 may be above and/or on top of lower portion 406 and be designed to accommodate an internal cavity. In at least one embodiment, the over-molding 405 may protrude about 2 mm - 6 mm, above the uppermost thread of lower portion 406, for example. In at least one embodiment, over molding 405 protrudes about 3.5 mm above lower portion 406, for example. In some embodiments, lower portion 406 may be formed of a metallic component, e.g. titanium and titanium alloys and include an external thread pattern on outside circumferential surface thereof, for example. As seen best in FIG. 9, an upper portion of the internal cavity 409 may be contained within over-molding 405 and a lower portion of the internal cavity 409 may be contained within lower portion 406 (as shown in FIG. 12). In various embodiments, a ceramic mold 407 may be inlayed within and/or contoured to an interior of over-molding 405. In various embodiments, ceramic mold 407 may be referred as a ceramic cap 407 and as explained in further detail below, there may be a thin cavity and/or gap space between the ceramic cap 407 and the over-molding 405. In this embodiment, the gap space may help to suppress and/or prevent potentially damaging internal flexural and shear stresses from being applied to the sensitive electronics equipment and hermetic seal. In various embodiments, ceramic cap 407 may be formed of Zirconia 3Y-TZP and/or a Zirconia toughed alumina (ZTA), for example.

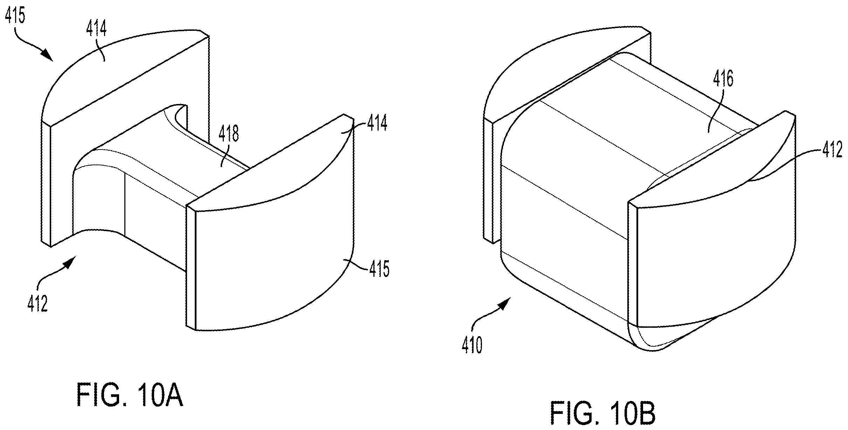

FIG. 10A illustrates a perspective view of a ferrite core 412 for use with digital set screw 400. Ferrite core 412 includes a bar or strut 418 coupled to and/or integrally formed with side portions 415. In at least one embodiment, the side portions 415 and strut 418 may be separately formed and assembled together. Side portions 415 may have an outermost curved surface generally conforming to and corresponding to a curvature of cavity 409, for example. Additionally, ferrite core 412 includes uppermost surfaces 414 that are planar and/or substantially planar. In other embodiments, uppermost surfaces 414 may be arcuate and/or curved to closely follow the inner profile of the cavity and/or ceramic cap. In the embodiment of FIG. 10A, an open area or void space is formed between side portions 415 and strut 418. As shown in FIG. 10B, the void space is used as a winding area for winding of a wire into a coil, e.g., a copper wire is wound around strut 418 between the internal side surfaces of side portions 415 thereby forming an assembled antenna 410. In the disclosed embodiment, the windings may be considered to be oriented in a lateral direction with respect to a longitudinal axis of the digital set screw 400, for example. In various embodiments, such copper windings may have a relatively consistent and thin diameter or copper windings may be ribbon like and relatively thin. Other materials than copper may also be used, for example silver, gold, stainless steel alloys and various combinations of conductive materials.

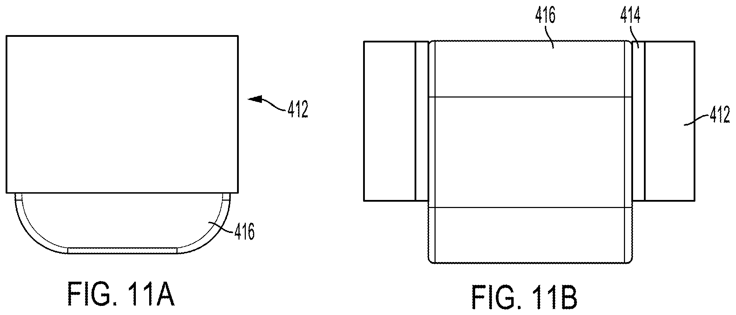

FIG. 11A illustrates a first side view of the antenna 410 of FIG. 10B and FIG. 11B illustrates a second side view of the antenna of FIG. 10B. In the illustrated embodiment, the windings 416 are flush with the upper surfaces 414 of ferrite core 412. The inventors have discovered that, at least in some embodiments, an antenna 410 formed of a ferrite core 412 with windings 416 spooled around strut 418 to such a relative height that windings 416 are substantially flush with uppermost surfaces 414 is a highly advantageous design. For example, this antenna design is well suited to the size constraints and limitations of the relatively small cavity 409 while also still having relatively strong transmission abilities, a known issue with various digital set screw embodiments. An additional factor may be in an ability for a digital set screw to couple to a power unit and/or receive a wireless charge as a transmission from an external device. Various embodiments disclosed herein have been shown by experimental testing to have a greater transmission ability for receiving and sending as compared to other antenna designs, e.g., such as longitudinal coil designs.

FIG. 12 illustrates a cross section view of set screw 400. This embodiment may include the same, similar, and/or substantially the same features as the embodiment of FIG. 9. This embodiment may differ from the embodiment of FIG. 9 in that an overmold portion 405 separates a first cavity 409 and a second cavity 411. The first cavity 409 may house the antenna, 410 while the second cavity 411 may house the sensor(s) 430, for example. Additionally, in various embodiments, microelectronics 420 (not illustrated in FIG. 12) may be disposed in either of (or both) the first cavity 409 and second cavity 411. In the cross-section view, it is shown that the windings 416 are disposed above and below strut 418 and between side portions 415. It is also shown that ferrite core 412, in cross section, has a substantially U shape. Sensors 430 may be disposed on the uppermost internal surface 452 of cover 450, for example on an opposite surface from the exposed lower most surface 451 of cover 450. In at least some embodiments, cover 450 may be formed of a metallic material, e.g., titanium and titanium alloys and may be formed of the same material as the other components of the threaded body. In at least one embodiment, the threaded body of digital set screw 400 and cover 450 may be formed of Ti-6AL-4V, for example. In various embodiments, lower most surface 451 may comprise a dimple as shown in FIG. 12. In various embodiments, a dimple of lower most surface 451 may directly contact a spinal implant, e.g., a spinal support rod and facilitate retaining the spinal implant in a particular position.

FIG. 13 shows a remaining portion 402 of a digital set screw 400 after a breakoff portion 401 has been removed. In the example embodiment, a head portion 408 of the remaining portion may be driven and/or rotated by driver, e.g., a torx T50 driver, a hexalobe driver, etc. In the example embodiment, a slit extends through the uppermost surface of head portion 408 and on opposing side surfaces which may be filled with overmold 405. At least one advantage of the slit and overmold 405 may be a relative increase in the efficacy of the power and communications of the antenna 410 on account of the removal of metal in this area. An additional advantage of the configuration shown in FIG. 13, may be that sharp edges associated with a breakoff location are reduced and/or eliminated. Additionally, the overmold 405 may be disposed between and/or offset from drive edges 408A to prevent drive stresses from cracking and/or adversely affecting overmold 405, for example. Furthermore, consistent with the disclosure herein, it shall be understood that FIG. 13 may also represent a digital set screw 400 having a lower profile which did not originally include a breakoff portion 401, for example.

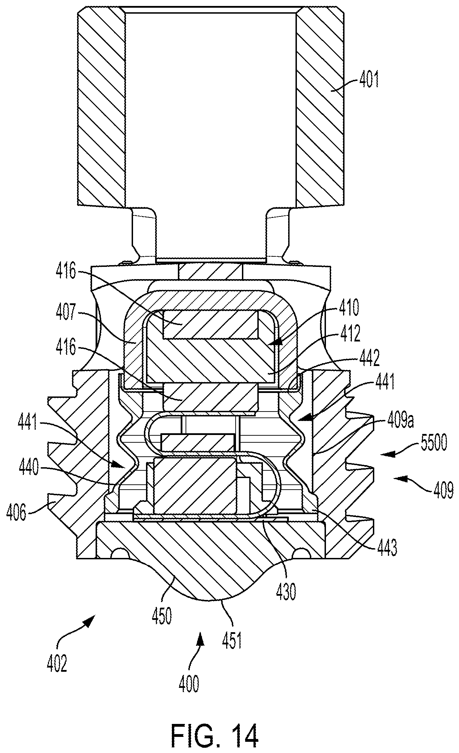

FIG. 14 illustrates a cross section drawing of another digital set screw 400 with an overmold portion 405 removed for ease of understanding. This embodiment may include the same, similar, and/or substantially the same features and functionality as the embodiment of FIG. 9 and the embodiment of FIG. 12. Accordingly, duplicative description will be omitted. This embodiment may differ in that the antenna 410 and ceramic mold 407 are supported on a bellows 440, for example. In this embodiment, bellows 440 may be flexible and serve a purpose of mechanically isolating various at-risk components from internal forces that may be transmit through the digital set screw 400 upon driving it, for example ceramic mold 407. In this embodiment, bellows 440 facilitates the creation and maintenance of a hermetic seal and preservation of the integrity of antenna 410 and other electronic components 5500 within the hermetic seal, for example.

In various embodiments, bellows 440 may be formed of a relatively thin metallic material on the order of about 50um to about 150um, and in other embodiments bellows 440 may be about 100um thick. In the example embodiment, bellows 440 may have a shape resembling an accordion and be compressible and extendable in a longitudinal direction of digital set screw 400 while also allowing for flexural deflection in a lateral direction. However, in other embodiments it shall be understood that bellows 440 may have a shape resembling a leaf spring, a coil spring, and other similar structures. As illustrated, bellows 440 includes an upper support surface 442 and an optional sidewall surface immediately adjacent upper support surface 442 forming a channel with which ceramic mold 407 may rest. The ceramic mold may be securely coupled to bellows 440 by a braze joint 442 or by any type of suitable weld or adhesive, for example aspot weld, laser weld, sonic weld, and/or an epoxy. Additionally, bellows 440 may be securely coupled to an interior sidewall 409A of digital set screw 400 at weld location 443. In this way, bellows 440 is securely coupled to the interior cavity 409 of digital set screw 400 at a lower region only. However, in other embodiments bellows 440 may additionally or alternatively be secured to the interior sidewall 409A defining interior cavity 409 at an intermediate region and/or an upper region, for example. In another alternate embodiment, the bellows 440 may additionally or alternatively be secured to the cover 450. Furthermore, bellows 440 may have a size and shape such that a gap space 441 is formed between bellows 440 and interior sidewall 409A of digital set screw 400. Gap space 441 may be any size such that it allows sufficient room for bellows 440 to deflect under the expected loads digital set screw 400 may sustain in ordinary use. In some embodiments, the side walls 409A may also deflect and the gap may additionally serve the functional benefit of preventing the bellows 440 and/or ceramic from coming into contact with the side walls 409A (at least at regions where the bellows 440 and/or ceramic is not coupled to the wisewalls 409A). Consistent with the disclosure herein, and as an example configuration, bellows 440 is fully and/or substantially disposed beneath the windings 412 and therefore will not create any significant interference with the performance of antenna 410.

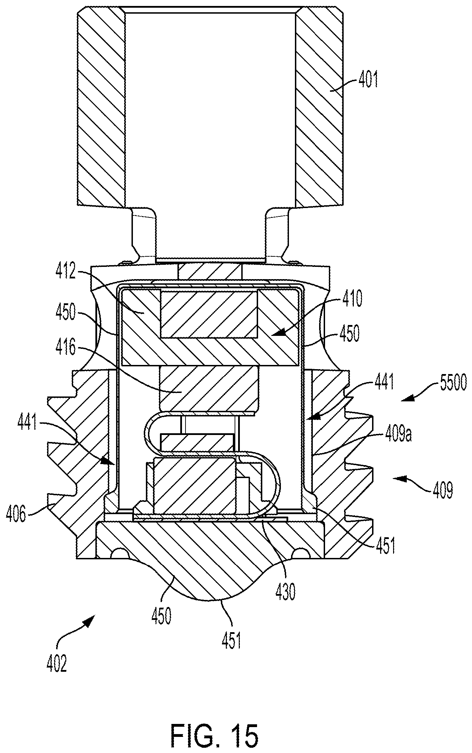

FIG. 15 illustrates a cross-section drawing of another digital set screw 400 with an overmold portion 405 removed for ease of understanding. This embodiment may include the same, similar, and/or substantially the same features and functionality as the embodiment of FIGS. 9, 12, and 14 for example. Accordingly, duplicative description will be omitted. This embodiment may differ in that the antenna 410 and various other electronics components 5500 are disposed within a cylindrical structure 450, for example. Cylindrical structure 450 may resemble a can having a closed upper surface, closed sidewall surfaces, and an open lower surface facing cover 450, for example. In various embodiments, cylindrical structure 450 be a relatively thin-walled structure on the order of about 25um to about 150um, and in some embodiments about 50um to about 100um depending on manufacturing tolerances. Cylindrical structure 450 may be formed of any metallic material or alloy, for example Ti-64 / Ti-811 and can serve at least one purpose of forming a hermetic enclosure surrounding antenna 410 and various other electronics components 5500, for example. In this embodiment, the ferrite core 412 of antenna 410 may be relatively larger than the embodiment shown in FIGS. 12 and 14 and be formed to have a cylindrical shape approximating the available space defined by cylindrical structure 450, for example. Similar to the embodiment shown in FIG. 14, the cylindrical structure may be disposed within cavity 409 and welded to an interior sidewall 409A of digital set screw 400.

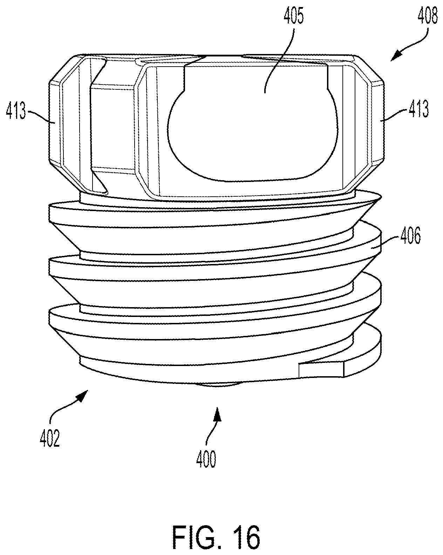

FIG. 16 illustrates a perspective view of a digital set screw 400. This embodiment illustrates a remaining portion 402 of a digital set screw 400 after a breakoff portion 401 has been removed. In the example embodiment, a head portion 408 of the remaining portion 402 may resemble a spline screw and may be driven and/or rotated by any suitable driver having a corresponding shape. In the example embodiment, a slit and/or opening extends through the body portion of digital set screw 400 in the uppermost surface of head portion 408 and on opposing side surfaces. The slit may separate the uppermost surface of head portion 408 into two parts and thereby prevent an eddy current from forming and interfering with the performance of the antenna and/or other electronics. In FIG. 16, the slit is shown as being filled with overmold 405, which may be an insulator formed of a thermoplastic. At least one advantage of the slit and overmold may be a relative increase in the performance of the antenna 410 on account of the removal of metal in this area. Another advantage of the configuration shown in FIG. 16, may be that sharp edges associated with a breakoff location are reduced and/or eliminated. In some embodiments, and as shown in FIG. 16, overmold portion 405 may also correspond spatially to the location of antenna 410 and thereby reduce interference associated with metallic body of digital set screw 400. Additionally, in various spline drive embodiments, drive edges 413 may be spaced around the outside circumferential sides of digital set screw 400 to assist with the distribution of localized forces to portion 402 to prevent drive stresses from cracking and/or adversely affecting overmold 405, for example. In one example, three drive edges 413 are disposed on each side of the slit and overmold 405 (total of six drive edges 413). In various embodiments, the drive edges 413 may be symmetrically spaced around the diameter of head portion 408 and or unevenly spaced around the diameter of head portion 408. In at least one embodiment, a first group of three drive edges are clustered on a first side of slit and overmold 405 and a second group of three drive edges are clustered on a second side of slit and overmold 405 in a non-symmetrical spacing such that drive edges are farther from the relatively weaker area of the set screw 400 corresponding to the slit and overmold 405. In various embodiments, this offset nature may improve performance and viability of the digital set screw 400 because torque may be applied in an efficient and distributed manner that prevents and/or suppresses the possibility of digital set screw 400 buckling at high loads along the portion of set screw 400 corresponding to slit and overmold 405. In at least one embodiment, experimental testing has shown that the embodiment of FIG. 16 is capable of enduring forces in excess of about 15Nm without buckling. Furthermore, consistent with the disclosure herein, it shall be understood that FIG. 16 may also represent a digital set screw 400 having a lower profile which did not originally include a breakoff portion 401, for example.

Further non-claimed embodiments of the invention are as follows:

There is provided a load sensing assembly (e.g., a load sensing system), the load sensing assembly (e.g., load sensing system) comprising: a plurality of set screws, each set screw comprising: an upper surface, an outside thread pattern disposed on a circumferential side surface, a bottom surface, and an internal cavity disposed between the bottom surface, the circumferential side surface, and the upper surface; an antenna comprising a ferrite core and a plurality of windings; and at least one sensor comprising an integrated circuit in communication with the antenna, the sensor configured to detect an external force applied to the bottom surface, wherein the sensor and integrated circuit are positioned within the internal cavity, and wherein the antenna is configured to transmit information received from the at least one sensor to an external device.

Further, there is provided the load sensing assembly of the first further embodiment, wherein each ferrite core comprises a strut disposed between opposite side portions and each of the plurality of windings are wound around the strut.

Further, there is provided the load sensing assembly of the second further embodiment, wherein each of the opposite side portions comprises a curved side surface having a size and shape generally corresponding to a size and shape of side surfaces of the internal cavity.

## Claims

1. A load sensing assembly for a spinal implant, the load sensing assembly comprising:
a set screw (400) comprising an upper surface, an outside thread pattern (406) disposed on a circumferential side surface, a bottom surface, and an internal cavity (409) disposed between the bottom surface, the circumferential side surface, and the upper surface;
an antenna (410); and
at least one sensor (430) comprising an integrated circuit in communication with the antenna (410), the sensor (430) configured to detect an external force applied to the bottom surface,
wherein the sensor (430) and the integrated circuit are positioned within the internal cavity (409), and
wherein the antenna (410) is configured to transmit information received from the at least one sensor (430) to an external device,
**characterized in that**:
the antenna (410) comprises a ferrite core (412) and a plurality of windings (416);
the ferrite core (412) comprises a strut (418) disposed between opposite side portions (415); and
the plurality of windings (416) are wound around the strut (418), and
an uppermost surface of the plurality of windings (416) is substantially flush with an uppermost elevation (414) of the ferrite core (412).

2. The load sensing assembly of claim 1, wherein each of the opposite side portions (415) comprises a curved side surface having a size and shape generally corresponding to a size and shape of side surfaces of the internal cavity (409).

3. The load sensing assembly of one of claims 1-2, wherein each winding (416) of the plurality of windings (416) is oriented in a lateral direction with respect to a longitudinal axis of the set screw (400).

4. The load sensing assembly of one of claims 1-3, wherein the internal cavity (409) is hermetically sealed.

5. The load sensing assembly of one of claims 1-4, further comprising a breakoff portion (401) coupled to the upper surface at a fracture location, wherein the upper surface comprises an over-mold (405) disposed between the fracture location and an uppermost thread of the outside thread pattern (406).

6. The load sensing assembly of one of claims 1-5, further comprising a breakoff portion (401) coupled to the upper surface at a fracture location, wherein the bottom surface is defined by a cover (450) having a dimple on a lowermost exposed surface (451), and the cover (450) is welded to a lower portion of the internal cavity (409).

7. The load sensing assembly of one of claims 1-6, further comprising a plurality of electronics components (5500) including the at least one sensor (430) and the integrated circuit.

8. The load sensing assembly of claim 7, wherein the set screw (400) further includes another sensor (430) that comprises one or more of the following: a strain gauge, impedance sensor, pressure sensor, and capacitive sensor.

9. The load sensing assembly of claim 7 or 8, wherein the at least one sensor (430) is operably connected to the cover (450) and configured to sense an external force applied to the dimple.

10. The load sensing assembly of one of claims 7-9, wherein the electronics components (5500) are powered by electromagnetic energy.

11. The load sensing assembly of one of claims 7-10, wherein the electronics components (5500) are powered by a battery.

12. The load sensing assembly of one of claims 1-11, wherein the at least one sensor (430) is configured to measure a force between the set screw (400) and a longitudinal member when the set screw (400) is engaged with an anchoring member.

13. The load sensing assembly of one of claims 1-12, further comprising:
a mechanical isolation component configured to mechanically isolate the antenna (410) and the at least one sensor (430) from internal forces applied to the set screw (400),
wherein a gap space is disposed between the mechanical isolation component and an interior sidewall of the internal cavity (409).

14. The load sensing assembly of one of claims 1-13, further comprising:
a bellows (440) disposed within the internal cavity (409) and selectively coupled to an interior sidewall (409A) of the internal cavity (409); and
a gap space (441) between the bellows (440) and the interior sidewall (409A),
wherein the antenna (410) is coupled to the bellows (440).

15. The load sensing assembly of one of claims 1-14, further comprising:
a cylindrical cap disposed within the internal cavity (409) and selectively coupled to an interior sidewall of the internal cavity (409), the cylindrical cap having an opening facing the bottom surface of the set screw (400); and
a gap space between the cylindrical cap and the interior sidewall,
wherein the antenna (410) is disposed within an upper region of the cylindrical cap, the antenna (410) comprising a size and shape that corresponds to a size and shape of an internal diameter of the cylindrical cap.

## Patentansprüche

1. Lasterfassungsanordnung für ein Wirbelsäulenimplantat, die Lasterfassungsanordnung umfassend:
eine Stellschraube (400), umfassend eine obere Oberfläche, ein auf einer umlaufenden Seitenoberfläche eingerichtetes Außengewindemuster (406), eine untere Oberfläche und einen zwischen der unteren Oberfläche, der umlaufenden Seitenoberfläche und der oberen Oberfläche eingerichteten Innenhohlraum (409);
eine Antenne (410); und
mindestens einen Sensor (430), umfassend einen integrierten Schaltkreis, der mit der Antenne (410) in Verbindung steht, wobei der Sensor (430) konfiguriert ist, um eine auf die untere Oberfläche ausgeübte äußere Kraft zu erkennen,
wobei der Sensor (430) und der integrierte Schaltkreis innerhalb des Innenhohlraums (409) positioniert sind, und
wobei die Antenne (410) konfiguriert ist, um von dem mindestens einen Sensor (430) empfangene Informationen an eine externe Vorrichtung zu übertragen,
**dadurch gekennzeichnet, dass:**
die Antenne (410) einen Ferritkern (412) und eine Vielzahl von Wicklungen (416) umfasst;
der Ferritkern (412) eine Strebe (418) umfasst, die zwischen gegenüberliegenden Seitenabschnitten (415) eingerichtet ist; und
die Vielzahl von Wicklungen (416) um die Strebe (418) gewickelt sind, und
eine oberste Oberfläche der Vielzahl von Wicklungen (416) im Wesentlichen bündig mit einer obersten Erhebung (414) des Ferritkerns (412) ist.

2. Lasterfassungsanordnung nach Anspruch 1, wobei jeder der gegenüberliegenden Seitenabschnitte (415) eine gekrümmte Seitenoberfläche umfasst, die eine Größe und eine Form im Wesentlichen entsprechend einer Größe und einer Form der Seitenoberflächen des Innenhohlraums (409) aufweist.

3. Lasterfassungsanordnung nach einem der Ansprüche 1 bis 2, wobei jede Wicklung (416) der Vielzahl von Wicklungen (416) in einer seitlichen Richtung in Bezug auf eine Längsachse der Stellschraube (400) ausgerichtet ist.

4. Lasterfassungsanordnung nach einem der Ansprüche 1 bis 3, wobei der Innenhohlraum (409) hermetisch abgedichtet ist.

5. Lasterfassungsanordnung nach einem der Ansprüche 1 bis 4, ferner umfassend einen Sollbruchabschnitt (401), der an einer Bruchstelle mit der oberen Oberfläche gekoppelt ist, wobei die obere Oberfläche eine Überformung (405) umfasst, die zwischen der Bruchstelle und einem obersten Gewinde des Außengewindemusters (406) eingerichtet ist.

6. Lasterfassungsanordnung nach einem der Ansprüche 1 bis 5, ferner umfassend einen Sollbruchabschnitt (401), der an einer Bruchstelle mit der oberen Oberfläche gekoppelt ist, wobei die untere Oberfläche durch eine Abdeckung (450) definiert ist, die an einer untersten freiliegenden Oberfläche (451) eine Vertiefung aufweist, und die Abdeckung (450) mit einem unteren Abschnitt des Innenhohlraums (409) verschweißt ist.

7. Lasterfassungsanordnung nach einem der Ansprüche 1 bis 6, ferner umfassend eine Vielzahl von elektronischen Komponenten (5500) einschließlich des mindestens einen Sensors (430) und des integrierten Schaltkreises.

8. Lasterfassungsanordnung nach Anspruch 7, wobei die Stellschraube (400) ferner einen weiteren Sensor (430) einschließt, der einen oder mehrere der Folgenden umfasst: einen Dehnungsmessstreifen, einen Impedanzsensor, einen Drucksensor und einen kapazitiven Sensor.

9. Lasterfassungsanordnung nach Anspruch 7 oder 8, wobei der mindestens eine Sensor (430) mit der Abdeckung (450) betriebsmäßig verbunden ist und konfiguriert ist, um eine auf die Vertiefung ausgeübte externe Kraft zu erfassen.

10. Lasterfassungsanordnung nach einem der Ansprüche 7 bis 9, wobei die elektronischen Komponenten (5500) durch elektromagnetische Energie angetrieben werden.

11. Lasterfassungsanordnung nach einem der Ansprüche 7 bis 10, wobei die elektronischen Komponenten (5500) durch eine Batterie angetrieben werden.

12. Lasterfassungsanordnung nach einem der Ansprüche 1 bis 11, wobei der mindestens eine Sensor (430) konfiguriert ist, um eine Kraft zwischen der Stellschraube (400) und einem Längselement zu messen, wenn die Stellschraube (400) mit einem Verankerungselement in Eingriff steht.

13. Lasterfassungsanordnung nach einem der Ansprüche 1 bis 12, ferner umfassend:
eine mechanische Isolationskomponente, die konfiguriert ist, um die Antenne (410) und den mindestens einen Sensor (430) von inneren Kräften, die auf die Stellschraube (400) ausgeübt werden, mechanisch zu isolieren,
wobei zwischen der mechanischen Isolationskomponente und einer inneren Seitenwand des Innenhohlraums (409) ein Spaltraum eingerichtet ist.

14. Lasterfassungsanordnung nach einem der Ansprüche 1 bis 13, ferner umfassend:
einen Balg (440), der innerhalb des Innenhohlraums (409) eingerichtet ist und mit einer inneren Seitenwand (409A) des Innenhohlraums (409) wahlweise gekoppelt ist; und
einen Spaltraum (441) zwischen dem Balg (440) und der inneren Seitenwand (409A),
wobei die Antenne (410) mit dem Balg (440) gekoppelt ist.

15. Lasterfassungsanordnung nach einem der Ansprüche 1 bis 14, ferner umfassend:
eine zylindrische Kappe, die innerhalb des Innenhohlraums (409) eingerichtet und mit einer inneren Seitenwand des Innenhohlraums (409) wahlweise gekoppelt ist, wobei die zylindrische Kappe eine Öffnung aufweist, die der unteren Oberfläche der Stellschraube (400) zugewandt ist; und
einen Spaltraum zwischen der zylindrischen Kappe und der inneren Seitenwand,
wobei die Antenne (410) in einem oberen Bereich der zylindrischen Kappe eingerichtet ist, die Antenne (410) umfassend eine Größe und eine Form, die einer Größe und einer Form eines Innendurchmessers der zylindrischen Kappe entspricht.

## Revendications

1. Ensemble de détection de charge pour un implant vertébral, l'ensemble de détection de charge comprenant :
une vis de réglage (400) comprenant une surface supérieure, un motif de filetage extérieur (406) disposé sur une surface latérale circonférentielle, une surface inférieure, et une cavité interne (409) disposée entre la surface inférieure, la surface latérale circonférentielle, et la surface supérieure ;
une antenne (410) ; et
au moins un capteur (430) comprenant un circuit intégré en communication avec l'antenne (410), le capteur (430) configuré pour détecter une force externe appliquée à la surface inférieure,
dans lequel le capteur (430) et le circuit intégré sont positionnés à l'intérieur de la cavité interne (409), et
dans lequel l'antenne (410) est configurée pour transmettre à un dispositif externe les informations reçues de l'au moins un capteur (430),
**caractérisé en ce que** :
l'antenne (410) comprend un noyau de ferrite (412) et une pluralité d'enroulements (416) ;
le noyau de ferrite (412) comprend une entretoise (418) disposée entre les parties latérales opposées (415) ; et
la pluralité d'enroulements (416) est enroulée autour de l'entretoise (418), et
une surface la plus élevée de la pluralité d'enroulements (416) est sensiblement au même niveau qu'une élévation la plus élevée (414) du noyau de ferrite (412).

2. Ensemble de détection de charge selon la revendication 1, dans lequel chacune des parties latérales opposées (415) comprend une surface latérale incurvée ayant une taille et une forme correspondant généralement à une taille et une forme des surfaces latérales de la cavité interne (409).

3. Ensemble de détection de charge selon l'une des revendications 1-2, dans lequel chaque enroulement (416) de la pluralité d'enroulements (416) est orienté dans une direction latérale par rapport à un axe longitudinal de la vis de réglage (400).

4. Ensemble de détection de charge selon l'une quelconque des revendications 1-3, dans lequel la cavité interne (409) est hermétiquement fermée.

5. Ensemble de détection de charge selon l'une des revendications 1-4, comprenant en outre une partie de rupture (401) accouplée à la surface supérieure au niveau d'un point de rupture, dans lequel la surface supérieure comprend un surmoulage (405) disposé entre le point de rupture et un fil le plus élevé du motif de filetage extérieur (406).

6. Ensemble de détection de charge selon l'une des revendications 1-5, comprenant en outre une partie de rupture (401) accouplée à la surface supérieure au niveau d'un point de rupture, dans lequel la surface inférieure est définie par un couvercle (450) ayant une alvéole sur une surface la plus basse exposée (451), et le couvercle (450) est soudé à une partie inférieure de la cavité interne (409).

7. Ensemble de détection de charge selon l'une des revendications 1-6, comprenant en outre une pluralité de composants électroniques (5500) comportant l'au moins un capteur (430) et le circuit intégré.

8. Ensemble de détection de charge selon la revendication 7, dans lequel la vis de réglage (400) comporte en outre un autre capteur (430) qui comprend un ou plusieurs des éléments suivants : une jauge de contrainte, un capteur d'impédance, un capteur de pression et un capteur capacitif.

9. Ensemble de détection de charge selon la revendication 7 ou 8, dans lequel l'au moins un capteur (430) est connecté de manière opérationnelle au couvercle (450) et configuré pour détecter une force externe appliquée à l'alvéole.

10. Ensemble de détection de charge selon l'une des revendications 7-9, dans lequel les composants électroniques (5500) sont alimentés par l'énergie électromagnétique.

11. Ensemble de détection de charge selon l'une des revendications 7-10, dans lequel les composants électroniques (5500) sont alimentés par une batterie.

12. Ensemble de détection de charge selon l'une des revendications 1-11, dans lequel l'au moins un capteur (430) est configuré pour mesurer une force entre la vis de réglage (400) et un élément longitudinal lorsque la vis de réglage (400) est en prise avec un élément d'ancrage.

13. Ensemble de détection de charge selon l'une des revendications 1-12, comprenant en outre :
un composant d'isolation mécanique conçu pour isoler mécaniquement l'antenne (410) et l'au moins un capteur (430) des forces internes appliquées à la vis de réglage (400),
dans lequel un espace vide est disposé entre le composant d'isolation mécanique et une paroi intérieure de la cavité interne (409).

14. Ensemble de détection de charge selon l'une des revendications 1-13, comprenant en outre :
un soufflet (440) disposé à l'intérieur de la cavité interne (409) et accouplé sélectivement à une paroi intérieure (409A) de la cavité interne (409) ; et
un espace vide (441) entre le soufflet (440) et la paroi intérieure (409A),
dans lequel l'antenne (410) est accouplée au soufflet (440).

15. Ensemble de détection de charge selon l'une des revendications 1-14, comprenant en outre :
un capuchon cylindrique disposé à l'intérieur de la cavité interne (409) et accouplé sélectivement à une paroi intérieure de la cavité interne (409), le capuchon cylindrique ayant une ouverture faisant face à la surface inférieure de la vis de réglage (400) ; et
un espace vide entre le bouchon cylindrique et la paroi intérieure,
dans lequel l'antenne (410) est disposée à l'intérieur d'une zone supérieure du bouchon cylindrique, l'antenne (410) comprenant une taille et une forme qui correspondent à une taille et une forme d'un diamètre interne du bouchon cylindrique.
